# EUROPÄISCHE PATENTSCHRIFT

(11) **EP 0 525 536 B1**
(45) Veröffentlichungstag und Bekanntmachung des Hinweises auf die Patenterteilung: **17.05.1995**
(21) Anmeldenummer: 92112200.8
(22) Anmeldetag: 17.07.1992
(51) Int. Cl.: C07C 213/08, C07C 217/42, C07C 217/40

(54) **Verfahren zur Herstellung von ortho-Amiden**
Process for the preparation of ortho-amides
Procédé pour la préparation d'ortho amides

(30) Priorität: 30.07.1991 DE 4125159
(43) Veröffentlichungstag der Anmeldung: 03.02.1993
(73) Patentinhaber: BAYER AG, 51368 Leverkusen (DE)
(72) Erfinder: Blank, Heinz-Ulrich, Dr., W-5068 Odenthal (DE); Kraus, Helmut, Dr., W-5000 Köln 80 (DE); Marzolph, Gerhard, Dr., W-5000 Köln 80 (DE); Müller, Nikolaus, Dr., W-4019 Monheim (DE)

(56) Entgegenhaltungen:
- DE-A- 2 023 429
- DE-A- 2 215 954
- DE-B- 1 161 285
- DE-B- 1 205 548
- DE-B- 1 212 069
- US-A- 3 239 534
- BULLETIN DE LA SOCIETE CHIMIQUE DE FRANCE Nr. 12, 1968, PARIS FR Seiten 4985 - 4990 CL. FEUGEAS ET AL. 'Préparation et propriétés de composés à structure trivalente mixte: dérivés diméthylamino-1 gemdialcoxy-1,1'
- CHEMISCHE BERICHTE. Bd. 104, Nr. 3, 1971, WEINHEIM DE Seiten 924 - 931 H. BREDERECK ET AL. 'Othoamide, XVI Synthese von O.N- und N.N-Acetalen der alpha-Keto-carbons urenitrile sowie von Iminoestern'
- CHEMISCHE BERICHTE. Bd. 101, Nr. 1, 1968, WEINHEIM DE Seiten 41 - 50 H. BREDERECK ET AL. 'S ureamid-Reaktionen, L; Orthoamide, I - Darstellung und Eigenschaften der Amidacetale und Aminalester'
- Houben-Weyl, Band VI/2, Stuttgart 1963, Seiten 5 bis 9

## Beschreibung

Die Erfindung betrifft die Herstellung von ortho-Amiden (Alkoxy-aminomethanen), zu denen die beiden Gruppen der Dialkoxy-dialkylaminomethane (DMF-Acetale) und Alkoxy- bis(dialkylamino)-methane (Aminalester) gehören; zur Herstellung werden hochaktive Alkoholatsuspensionen eingesetzt. DMF-Acetale und Aminalester sind reaktive C₁-Bausteine zur Aminomethylenierung von C-H-aciden Verbindungen. Diese aminomethylenierten Substanzen stellen wertvolle Zwischenprodukte dar zur Synthese von Heterocyclen, wie Indolen, Pyrimidinen, Pyridinen und Chinoloncarbonsäuren.

Zur Herstellung von ortho-Amiden kann man durch Umsetzung von ortho-Estern mit sekundären Aminen stufenweise Alkoxy- gegen Aminogruppen an einem sauren Katalysator ersetzen. Das Gleichgewicht liegt jedoch auf der Seite der ortho-Ester, ein gezielter Austausch von einer oder zwei Alkoxygruppen ist schwierig, so daß diese Methode hauptsächlich zur Darstellung von Trisaminomethanen benutzt wird (DE-OS 22 14 497).

Die Verwendung von Chloroform statt der ortho-Ester (Rec. Trav. Chem. Pays-Bas 88 (1969), 289; DE-AS 11 61 285) hat sich als wenig geeignet herausgestellt.

Eine selektive Herstellung von DMF-Acetalen und Aminalestern ist ferner durch Umsetzung von Alkoxyiminomethylen- bzw. Formamidinium-Salzen mit Alkoholaten möglich (Chem. Ber. 101 (1968), 41). Die Ausbeuten liegen bei fünf verschiedenen Aminalestern zwischen 62 und 72 % und bei einem Aminalester bei 79 %; bei 14 verschiedenen Amidacetalen liegen sie zwischen 42 und 63 %, während das DMF-dimethylacetal in 75-87 % der theoretischen Ausbeute erhältlich sein soll. In DD 94 359 wird jedoch berichtet, daß das DMF-dimethylacetal nach diesem Verfahren wegen Zersetzung in maximal 50-55 % erhältlich sein soll. Gemäß Beschreibung in Helv. Chim. Acta 48 (1965), 1746 erhält man nach dieser Methode das DMF-dimethylacetal in einer Ausbeute von nur 37 % der theoretischen Ausbeute.

In DE-AS 12 05 548 wird die Herstellung verschiedener Aminalester beschrieben. Ausbeuten von 62-77 % werden unter Verwendung von 10 % Überschuß an teurem alkoholfreiem Alkoholat erhalten. Die Angabe, daß man ausgehend von rohem Formamidiniumsalz, das herstellungsbedingt ein Äquivalent Methanol enthält, in 68 % der theoretischen Ausbeute den Methylaminalester erhält, ließ sich nicht bestätigen. Bei einer Nacharbeitung fiel ein 1,8:1-Amidacetal-Aminalester-Gemisch in 65 % der theoretischen Ausbeute an.

Ohne Verwendung eines Lösungsmittels wurden nach US 3.239.534 aus Dimethylformamid, Dimethylsulfat und Alkoholat 19,1 % der theoretischen Ausbeute an DMF-Acetal und unter zusätzlicher Verwendung von Dimethylamin 25,6 % Aminalester erhalten.

Als eine elegante, da homogen ablaufende Reaktion wird die Umsetzung von Bis(dialkylamino)-acetonitrilen mit Alkoholaten beschrieben (Chem. Ber. 105 (1972), 1340). Die Ausbeuten an Aminalestern liegen bei 76-84 %, die Herstellung der verwendeten Acetonitrile ist jedoch nur in 53 % der theoretischen Ausbeute, ausgehend von den Formamidiniumsalzen, möglich (Chem. Ber. 104 (1971), 924).

Die DMF-Acetal-Synthese ist auch durch Umsetzung von zwei Äquivalenten Alkoholat mit Amidchloriden möglich (Angew. Chem. 72 (1969), 836: BE 598 238). Bei deren Herstellung aus Dialkylformamiden mit Chlorierungsmitteln, wie POCl₃, SOCl₂ u.ä., werden jedoch immer als Nebenprodukte die carcinogenen Carbamidchloride gebildet.

Neben den schwankenden und für technische Synthesen relativ geringen Ausbeuten von 40-75 % ist die literaturbekannte Aufarbeitung für eine industrielle Durchführung ungeeignet. Destilliert man nach der Umsetzung zum ortho-Amid zunächst das Lösungsmittel und anschließend das gewünschte Produkt vom Salzrückstand ab, so kommt es laut DD 94 359 zu Zersetzungen und Ausbeuteverlusten. Diese Patentschrift versucht daher, eine Ausbeutesteigerung auf 85 % durch Herausschleppen des Produktes mit Methanol zu erreichen. Man muß hierbei jedoch große Lösungsmittelmengen zweimal destillieren. Ferner ist die destillative Trennung von Methanol und DMF-dimethylacetal schwierig. Bei einer Nacharbeitung ließ sich außerdem die angegebene hohe Ausbeute nicht erreichen.

Die vorherige Abtrennung der Salze ist ebenfalls problematisch, Einerseits lösen sich Methylsulfate teilweise in polaren Medien, wie beispielsweise Alkoholen, andererseits fallen sie je nach Lösungsmittel und Durchführung der Umsetzung in gelartiger, klumpiger oder sehr feinkristalliner Form an, wodurch eine Filtration sehr schwierig wird.

Eine wäßrige Aufarbeitung ist wegen der Hydrolyseempfindlichkeit der ortho-Amide nicht durchführbar.

Überraschenderweise wurde nun ein Verfahren zur Herstellung von Amidacetalen und Aminalestern gefunden, welches diese bekannten Schwierigkeiten umgeht und in hoher, reproduzierbarer Ausbeute die ortho-Amide liefert. Bei diesem erfindungsgemäßen Verfahren wird ein Alkoholat in einer hochreaktiven Form suspendiert eingesetzt, da sich gezeigt hat, daß es nicht ausreicht, das Alkoholat in eine alkoholfreie Form zu bringen, wie es beispielsweise in Chem. Ber. 101 (1968), 41-50 oder in "Methoden der Organischen Chemie" (Houben-Weyl), Bd. VI/2, 4. Aufl., Georg Verlag, Stuttgart 1963, S. 5-9 beschrieben ist. Dort werden die Alkohole durch Eindampfen der alkoholischen Lösung zur Trockne (Vakuum, Inertgasstrom oder azeotrope Destillation) hergestellt und nach Aufschäumung in Ether oder Hexan umgesetzt.

Die Erfindung betrifft demnach ein Verfahren zur Herstellung von ortho-Amiden der Formel 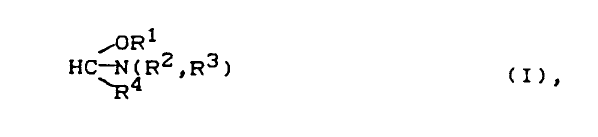
in der
- R¹: geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₂-C₈-Alkylen-OM¹ oder C₇-C₁₀-Aralkyl bedeutet,
- R² und R³: unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ gemeinsam mit dem N-Atom, das sie substituieren, einen 5- bis 8-gliedrigen Ring bilden können, der ein weiteres Heteroatom aus der Gruppe N, O und S enthalten kann, und
- R⁴: für -OR⁵ oder -N(R⁵,R⁶) steht, worin R⁵ und R⁶ unabhängig voneinander und unabhängig von R² und R³ den für R² und R³ genannten Bedeutungsumfang haben,
das dadurch gekennzeichnet ist, daß man Salze der Formel 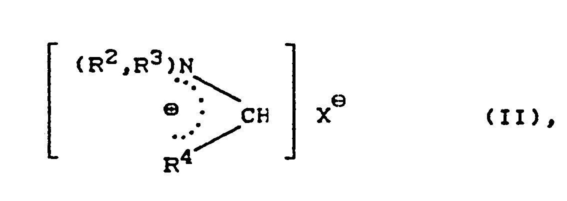
in der
- R², R³ und R⁴: die genannte Bedeutung haben und
- X^{⊖}: das C₁-C₈-Alkylsulfatanion, das C₆-C₁₂-Arylsulfonatanion, das Tetrafluoroboranatanion, das C₆-C₁₂-Arylsulfatanion, das Chloridanion, das Bromidanion, das Iodidanion, das Hexafluorophosphatanion, das C₁-C₈-Alkylsulfonatanion, das C₁-C₈-Halogenalkylsulfonatanion, das C₁-C₈-Halogenalkylsulfatanion, das Perchloratanion oder das Hexachloroantimonatanion bedeutet,
in Gegenwart von Alkoholaten der Formel

M¹OR¹ (III),

in der
- R¹: den genannten Bedeutungsumfang besitzt und
- M¹: ein Äquivalent eines Alkali- oder Erdalkalimetallkations ist,
in einem Nichtlösungsmittel umsetzt, wobei eine Alkoholatsuspension in demselben Nichtlösungsmittel eingesetzt wird, die durch Behandlung von in einem Alkohol gelösten Alkoholat in höhersiedenden Nicht-Lösungsmitteln bei einer Temperatur von 120 bis 200°C, bevorzugt 130 bis 180°C und destillativer Entfernung des gesamten Alkohols und Kristallalkohols erhalten worden ist.

Geradkettiges oder verzweigtes C₁-C₈-Alkyl ist beispielsweise Methyl, Ethyl, Propyl, Isopropyl, Butyl, Isobutyl, tert.-Butyl, die isomeren Amyle, Hexyle, Octyle, bevorzugt die genannten C₁-C₄-Alkylreste.

C₂-C₈-Alkenyl ist Vinyl, Propenyl, Allyl, die isomeren Butenyle, Amylenyle, Hexenyle oder Octenyle, bevorzugt die genannten C₃-C₄-Alkenylreste.

C₂-C₈-Alkoxyalkyl ist beispielsweise Methoxymethyl, Ethoxymethyl, Methoxyethyl sowie weitere Reste aus der Gruppe C₃-C₉-Alkyl, in welchem eine CH₂-Gruppe durch ein O-Atom ersetzt ist,

C₃-C₈-Alkoxyalkenyl ist beispielsweise Methoxyvinyl, Ethoxyvinyl, Methoxyallyl, 2-Methoxy-propenyl und andere aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methylcyclopropyl, Dimethyl-cyclopropyl, Cyclobutyl, Methylcyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate,

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, Morpholin, Pyran, Azepin, Azocin, Isoxazol, Isothiazol, Pyridazin und Pyrazin. Es ist dem Fachmann bekannt, daß ungesättigte heterocyclische Ringe einen mehr oder weniger stark ausgeprägten aromatischen Charakter haben können.

Weiterhin können R² und R³ gemeinsam mit dem N-Atom, das sie substituieren, einen 5- bis 8-gliedrigen gesättigten oder ungesättigten Ring bilden, der ein weiteres Heteroatom aus der Gruppe N, O und S enthalten kann. Solche Systeme sind beispielsweise Pyrrol, Pyrrolidin, Pyrrolin, Pyrazol, Pyrazolidin, Imidazol, Imidazolidin, Thiazol, Thiazolidin, Piperazin, Piperidin, Morpholin, Azepin, Dihydroazocin.

Für den Fall, daß R¹ für C₂-C₈-Alkylen-OM¹ steht, handelt es sich bei (III) um das Alkoholat eines Diols mit 2 bis 8 C-Atomen, wie Glykol, 1,2-Propandiol, 1,3-Propandiol, 1,2-, 1,3- oder 1,4-Butandiol, Hexandiol oder Octandiol.

Das C₁-C₈-Alkylsulfatanion ist beispielsweise das Anion von Methylschwefelsäure, Ethylschwefelsäure, Propylschwefelsäure, Isopropylschwefelsäure, Butylschwefelsäure, Isobutylschwefelsäure, eine der isomeren Hexylschwefelsäuren oder Octylschwefelsäuren.

Das C₁-C₈-Alkylsulfonatanion bzw. das C₁-C₈-Halogenalkylsulfonatanion ist beispielsweise das Anion der Methylsulfonsäure, der Trichlormethylsulfonsäure, der Trifluormethylsulfonsäure oder einer Sulfonsäure mit höherem (Halogen)Alkylrest.

Das C₆-C₁₂-Arylsulfonatanion ist beispielsweise das Anion von Benzolsulfonsäure, Naphthalin-sulfonsäure, Biphenyl-sulfonsäure, Toluolsulfonsäure, bevorzugt von Benzolsulfonsäure oder Toluolsulfonsäure.

Das C₆-C₁₂-Arylsulfatanion ist beispielsweise das Anion der Phenylschwefelsäure, der Naphthylschwefelsäure oder der Biphenylschwefelsäure.

X^{⊖} ist bevorzugt Halogenid oder Alkylsulfat, besonders bevorzugt Chlorid oder Methylsulfat.

M¹ ist ein Äquivalent eines Alkalimetall- oder Erdalkalimetallkations, beispielsweise des Kations von Lithium, Natrium, Kalium, Rubidium, Cäsium, Magnesium, Calcium, Strontium, Barium; bevorzugt das Kation eines Alkalimetalls, besonders bevorzugt das Kation von Natrium oder Kalium.

Unter die zur erfindungsgemäßen Umsetzung benutzten Salze fallen sowohl Alkoxymethyleniminiumsalze als auch Formamidiniumsalze der Formeln 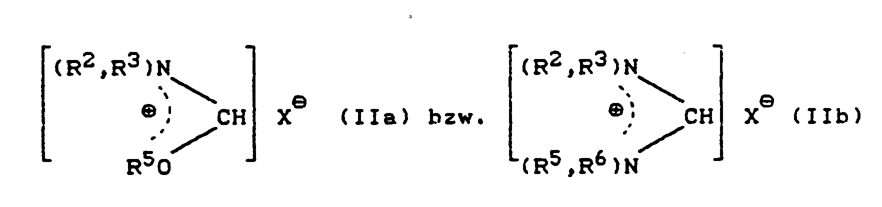
Die Reaktion des erfindungsgemäßen Verfahrens kann beispielhaft wie folgt formelmäßig dargestellt werden: 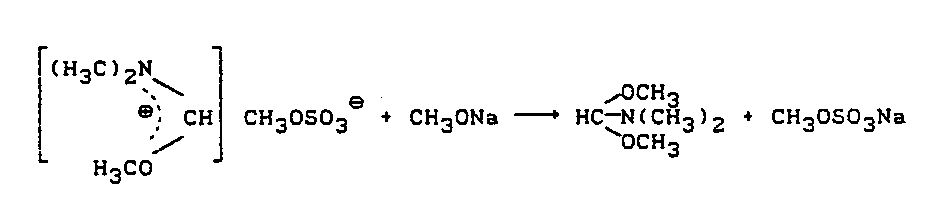
bzw. 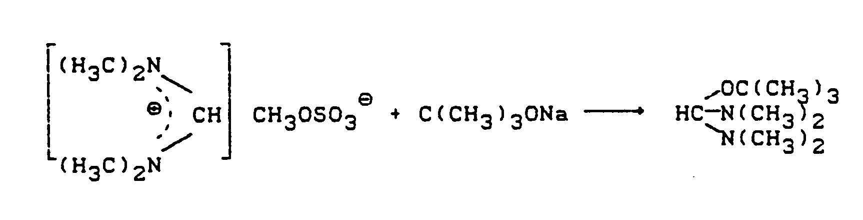
Für den Fall, daß R¹ und R⁵ nicht identisch sind, wird ein Acetal der Formel (I), in der also R⁴ = OR⁵ ist, als Gemisch der drei möglichen Acetale (R¹,R¹; R¹,R⁵; R⁵,R⁵) vorliegen. Aus einem solchen Gemisch kann durch Umacetalisierung eines der reinen Acetale gewonnen werden.

In bevorzugter Weise wird ein Salz der Formel 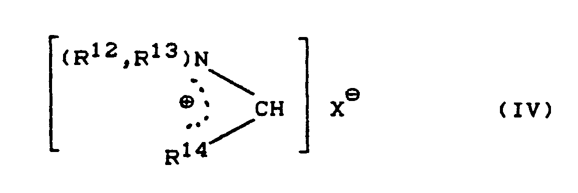
umgesetzt, in der
- R¹² und R¹³: unabhängig voneinander geradkettiges oder verzweigtes C₁-C₈-Alkyl, Cyclopropyl, Cyclopentyl, Cyclohexyl, Phenyl oder Benzyl bedeuten und R¹² und R¹³ weiterhin gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann,
- R¹⁴: für -OR¹² oder -N(R¹²,R¹³) steht und
- X^{⊖}: die obige Bedeutung annimmt.

In besonders bevorzugter Weise werden Salze der Formel 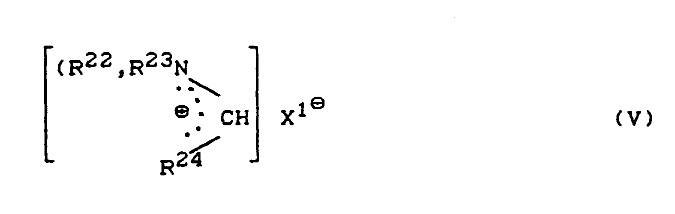
umgesetzt, in der
- R²² und R²³: unabhängig voneinander geradkettiges oder verzweigtes C₁-C₄-Alkyl bedeuten und R²² und R²³ weiterhin gemeinsam mit dem N-Atom, das sie substituieren, Morpholino, Pyrrolidino oder Piperidino bedeuten können,
- R²⁴: für -OR²² oder -N(R²²,R²³) steht und
- X^{1⊖}: das C₁-C₈-Alkylsulfatanion, das Chloridanion, das Bromidanion oder das Iodidanion bedeutet.

In ganz besonders bevorzugter Weise sind die Reste R⁵ und R⁶ identisch mit den Resten R² und R³. In weiterhin ganz besonders bevorzugter Weise bedeuten die Substituenten R², R³, R⁵ und R⁶ den Methylrest oder Ethylrest. In weiterhin bevorzugter Weise bedeutet X^{1⊖} das Chloridion oder das C₁-C₄-Sulfatanion, ganz besonders bevorzugt das Methylsulfatanion.

Das erfindungsgemäße Verfahren ist weiterhin besonders wichtig für Salze der Formel 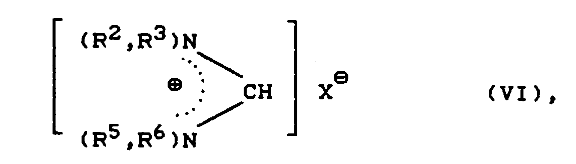
bevorzugt für Salze der Formel 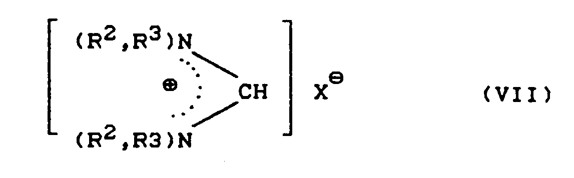
in denen
R², R³, R⁵, R⁶ und X^{⊖} die oben angegebene Bedeutung haben.

In weiterhin bevorzugter Weise wird die Umsetzung mit einem Alkoholat der Formel

M²OR¹¹ (VIII)

durchgeführt, in der
- R¹¹: für geradkettiges oder verzweigtes C₁-C₅-Alkyl, C₂-C₅-Alkoxyalkyl oder C₂-C₄-Alkylen-OM² steht und
- M²: Na^{⊕} oder K^{⊕} bedeutet.

In weiterhin besonders bevorzugter Weise wird die Umsetzung mit einem Alkoholat der Formel

M²OR²¹ (IX)

durchgeführt, in der
- R²¹: für geradkettiges oder verzweigtes C₁-C₅-Alkyl steht und
- M²: Na^{⊕} oder K^{⊕} bedeutet.

Als Lösungsmittel für das erfindungsgemäße Verfahren kommen inerte Lösungsmittel, wie (Halogen)Kohlenwasserstoffe, Ether u.a. zum Einsatz, die bevorzugt einen erhöhten Siedepunkt haben und weiter unten im einzelnen aufgeführt werden.

Es ist ein besonderes Kennzeichen der Erfindung, daß man ein hochaktives Alkoholat einsetzt. Es hat sich nämlich gezeigt, daß ein Alkoholat, das einem frisch geöffneten Versandgebinde entnommen wurde, nur unzureichende Ergebnisse bringt. Auch ein Alkoholat, dem durch zusätzliches Trocknen oder durch kurzes trockenes Andestillieren restlicher Alkohol, vor allem Kristallalkohol, entzogen Wurde, ergab keine wesentlich verbesserten Ergebnisse. Es hat sich jedoch überraschend gezeigt, daß ein feinverteiltes Alkoholat, das aus einer Lösung, beispielsweise einer alkoholischen Lösung, in Abwesenheit von Wasser, CO₂ und anderen mit Alkoholat reagierenden Stoffen gewonnen wurde, eine so hohe Aktivierung aufweist, daß eine sprunghafte Verbesserung der Ausbeute erreicht wird. Diese hohe Aktivierung steht in Übereinstimmung mit der Vorstellung, daß Alkoholate der oben genannten Herkunft eine oberflächliche Verkrustung durch Hydroxid oder Carbonat/Hydrogencarbonat aufweisen, die in heterogener Reaktion sehr hinderlich sind.

Die anspruchesgemäße Herstellungsmöglichkeit für ein hochaktives Alkoholat besteht darin, das in einem Alkohol gelöste Alkoholat in einem Nicht-Lösungsmittel auf eine Temperatur oberhalb der Abspaltungstemperatur des Kristallalkohols zu erhitzen. Hierzu muß dieses Nicht-Lösungsmittel einen Siedepunkt haben, der genügend weit oberhalb der Abspaltungstemperatur liegt, was auch durch Druckanwendung erreicht werden kann. Aus Gründen der leichteren Durchführbarkeit wird man jedoch Nicht-Lösungsmittel verwenden, deren Siedepunkt auch bei Normaldruck oberhalb von 120°C, bevorzugt oberhalb von 130°C liegt. Der abzutrennende Alkohol und Kristallalkohol wird sodann fraktioniert aus einem solchen erhitzten Gemisch oder als Gemisch oder Azeotrop mit dem eingesetzten hochsiedenden Nicht-Lösungsmittel herausdestilliert. Nicht-Lösungsmittel die sich hierzu besonders eignen sind: inerte, bevorzugt hochsiedende Flüssigkeiten, wie geradkettige oder verzweigte höhere Kohlenwasserstoffe der aliphatischen, aromatischen oder araliphatischen Reihe, beispielsweise Decan, Isododecan, Biphenyl, Xylol, Naphthalin, Triisopropylbenzol, Diphenylmethan, technische Gemische von benzylierten Benzolen, wie das Wärmeträgeröl Marlotherm S oder Autin B, ferner technische Mineralölgemische: ferner Halogenverbindungen, Ether, Nitroverbindungen, Nitrile, wie Dichlorbenzol, Diphenylether, Ditolylether, Nitrobenzol, Benzonitril; schließlich anorganischorganische Flüssigkeiten wie Silikonöl. Unter höherem Druck kommen auch sonst weniger hoch siedende Flüssigkeiten, wie Toluol, Cyclohexan, Petrolether, Dioxan, Tetrahydrofuran, Dibutylether und ähnliche in Betracht.

Solche erfindungsgemäß brauchbare, hochaktive Alkoholate weisen den zusätzlichen, aber nicht ausschlaggebenden Effekt auf, daß sie weitgehend alkoholfrei sind und demzufolge einen hohen Effektivgehalt haben (98 bis 100 % M¹OR¹).

Der überraschende Vorteil des erfindungsgemäßen Verfahrens wird deutlich, wenn man im Vergleich zur erfindungsgemäßen Verwendung eines Alkoholats in weitgehend alkoholfreier Form ein sonst technisch verfügbares festes Alkoholatpulver einsetzt, das einen Effektivgehalt im Bereich von etwa 90-97 % aufweist. Selbst wenn man unter sonst gleichen Bedingungen, d.h. auch in einer Suspension eines solchen technischen Alkoholatpulvers im vergleichbaren, höhersiedenden Lösungsmittel, arbeitet, werden Ausbeuten von lediglich 60-70 % der theoretischen Ausbeute erzielt.

Überraschenderweise wird erfindungsgemäß eine um bis zu 50 % höhere Ausbeute an gewünschtem ortho-Amid erreicht.

Die bevorzugte Variante der Herstellung eines weitgehend alkoholfreien Alkoholats durch Behandlung eines technischen Alkoholats in einem hochsiedenden Lösungsmittel und Fortführung der Reaktion des erfindungsgemäßen Verfahrens in der dabei erhaltenen Suspension des Alkoholats in diesem Lösungsmittel bedeutet zugleich die Vermeidung des Umgangs mit pulverförmigem Alkoholat, welches staubt, ätzend wirkt und an der Luft selbstentzündlich ist. Außerdem können bei dieser Form des Umgangs technisch verfügbare Alkoholatlösungen in Alkohol als Ausgangsmaterial verwendet werden, wie sie aus der Herstellung eines solchen Alkoholats anfallen. Dieser Vorteil gilt in besonderer Form für die niederen C₁-C₅-Alkoholate, und ganz besonders für den Einsatz von Methylat.

Die Durchführung des erfindungsgemäßen Verfahrens kann so erfolgen, daß man das Alkoxy-methyleniminium- bzw. Formamidinium-Salz aufschmilzt, in einem Lösungsmittel löst oder suspendiert und bei einer Temperatur von 0-80°C, bevorzugt 20-60°C zur Alkoholat-Suspension zudosiert. Auch die umgekehrte Zugabe ist möglich. Das Verhältnis Salz : Alkoholat liegt im Bereich von 1,5:1-1:1,5, bevorzugt im Bereich von 1,2:1-1:1,2. Nach einer kurzen Nachrührzeit ist die Reaktion beendet, Die Aufarbeitung gestaltet sich bei Verwendung eines der genannten inerten, hochsiedenden Lösungsmittel oder einem Gemisch mehrer von ihnen vorteilhaft. So werden die gewünschten ortho-Amide aus dem Reaktionsgemisch, in welchem das entstandene Salz in suspendierter Form vorliegt, durch Abdestillieren in reiner Form erhalten.

Durch einfaches Verwässern des Destillationsrückstandes und anschließende Phasentrennung wird das unpolare, hochsiedende Lösungsmittel nahezu vollständig zurückgeführt und kann nach kurzem Andestillieren zur Entfernung des darin vorhandenen Wassers wieder eingesetzt werden.

Die gewünschten ortho-Amide werden in reiner Form (Reinheit über 95 %, in den meisten Fällen über 97 %) erhalten. Als reine Form wird auch das Dismutierungsgemisch der ortho-Amide, speziell der Aminalester, verstanden, das im Verhaltnis Amidacetal : Aminalester : Trisdialkylaminomethan = 0:1:0 bis 0,33:0,34:0,33 anfallen kann. Da jedoch bei weiteren Umsetzungen, speziell mit Aminalestern, die Dismutierung schneller abläuft als diese weitere Reaktion, ist es unerheblich, welches Dismutierungsgemisch erfindungsgemäß als Reaktionsprodukt erhalten und dann für solche weiteren Reaktionen eingesetzt wird.

### Beispiele

(alle Operationen wurden unter Schutzgas durchgeführt)

### Beispiel 1

180 g 30 %ige methanolische Natriummethylatlösung und 250 ml Triisopropylbenzol wurden vorgelegt und erhitzt: bis 140°C Sumpftemperatur wurde Methanol abdestilliert. Bei 40°C tropfte man 221 g 96,1 %iges Tetramethylformamidiniummethylsulfat zu (enthielt 1,1 % DMF und 2,8 % Salz aus Dimethylamin und Methylschwefelsäure). Nach 20 Minuten Nachrühren wurde unter Verwendung einer Vigreux-Kolonne das Produktgemisch abdestilliert. Man erhielt 128 g 97,1 %igen Methylaminalester (2,8 % DMF; 0,1 % Triisopropylbenzol), der zu 15 % dismutiert war, entsprechend 94,1 % d.Th.. Der Destillationsrückstand wurde mit Wasser versetzt. Nach Phasentrennung wurde das organische Lösungsmittel zur Wasserentfernung andestilliert und für weitere Umsetzungen verwendet.

### Beispiel 2

Aus 324 g 21 %iger ethanolischer Natriumethylatlösung und 250 ml Triisopropylbenzol wurde analog Beispiel 1 eine Suspension hergestellt. Bei 40°C tropfte man 221 g 96,1 %iges Tetramethylformamidinium-methylsulfat zu. Nach der Destillation wurden 138 g 97,6 %iger Ethylaminalester (Rest DMF) erhalten (92,3 % d.Th.).

### Beispiel 3

274 g einer 35 %igen Natrium-tert.-butylat-Lösung in Tetrahydrofuran wurden mit 300 ml Mineralöl versetzt und der Ether destillativ entfernt. Bei 35°C tropfte man 230 g 96,1 %iges Tetramethylformamidinium-methylsulfat zu. Nach der Destillation erhielt man 163,8 g 96,7 %igen t-Butylaminalester als 0,24:0,48:0,28-Dismutierungsgemisch.

### Beispiel 4

55 g einer Schmelze aus 35 % Natriumisobutylat und 65 % Isobutanol Wurden mit 100 ml Mineralöl versetzt und der Alkohol destillativ entfernt. Bei 50°C tropfte man 44,6 g 95 %iges Tetramethylformamidinium-methylsulfat zu und erhielt nach der Destillation in 90,9 % d.Th. Isobutylaminalester als Dismutierungsgemisch.

### Beispiel 5

Von 370 g Kalium-tert.-pentylat-Lösung, die man durch Auflösen von 0,5 mol Kalium in 4 mol t-Pentanol erhalten hatte, wurde die Hälfte des Alkohols sofort, der Rest nach Zugabe von 120 ml Ditolylether abdestilliert. Bei 37°C tropfte man 110 g 96 %iges Tetramethylformamidinium-methylsulfat zu und destillierte das Produkt im Vakuum. Es wurden 91,3 % d.Th. an t-Pentyl-aminalester erhalten.

### Beispiel 6

Zu einer in Beispiel 1 beschriebenen Natriummethylat-Suspension tropfte man bei Raumtemperatur 203 g 97,1 %iges Methoxymethyleniminium-methylsulfat (enthielt 0,7 % Dimethylsulfat und 2,2 % DMF). Nach 30 min Nachrühren wurde das Produkt abdestilliert. Man erhielt in 92,4 % d.Th. 98,4 %iges DMF-Acetal (enthielt 0,9 % Methanol; 0,7 % DMF).

### Beispiel 7

Analog Beispiel 6 wurde Isododecan statt Triisopropylbenzol verwendet. Man erhielt in 90,4 % d.Th. DMF-Acetal.

### Vergleichsbeispiele

### Vergleichsbeispiel 1

180 g 30 %ige methanolische Natriummethylat-Lösung wurden mit 200 ml trockenem Methanol versetzt. Unter Eiskühlung tropfte man 205 g 91,1 %iges Methoxymethyleniminium-methylsulfat zu. Nach 1 h Nachrühren filtrierte man mit Hilfe einer Schutzgasfritte vom Salz ab und destillierte das Filtrat über eine Vigreux-Kolonne. Es wurden 68,3 g 96,3 %iges DMF-Acetal (Rest Methanol, Spur DMF) erhalten.

### Vergleichsbeispiel 2

227 g 21 %ige Natriumethylat-Lösung wurden zur Trockene eingedampft und mit 250 ml getrocknetem Methyl-tert.-butylether versetzt: bei 40°C wurden 148,4 g 97,4 %iges Tetramethylformamidinium-methylsulfat zugetropft. Nach 1 h Nachrühren bei Raumtemperatur filtrierte man über eine Schutzgasfritte und wusch mit 50 ml trockenem Cyclohexan nach. Nach ¹H-NMR-spektroskopischer Analyse des Filtrats war Ethylaminalester (als Dismutierungsgemisch) in 51,1 % d.Th. vorhanden.

### Vergleichsbeispiel 3

36,6 g Natriumethylat-Pulver aus einem frisch geöffneten Gefäß wurden in 300 ml Isododecan vorgelegt. Bei 40°C tropfte man 108,6 g 97,6 %iges Tetramethylformamidinium-methylsulfat zu und rührte 4 h bei Raumtemperatur nach. Bis 50°C/2 mbar wurden alle flüchtigen Bestandteile in eine Kühlfalle kondensiert. Das Destillat enthielt 44,0 % dismutierten Ethylaminalester, entsprechend 65,0 % d.Th..

### Vergleichsbeispiel 4

37,4 g technisches Natriumethylat-Pulver (90,9 %ig) wurden in 250 ml Triisopropylbenzol vorgelegt. Man erhitzte auf 140°C und destillierte durch Druckerniedrigung bis 100 mbar 30 ml Lösungsmittel ab. Anschließend wurden bei 40°C 108,6 g 97,6 %iges Tetramethylformamidiniummethylsulfat zugetropft und analog Vergleichsbeispiel 3 aufgearbeitet. Man erhielt in 68,3 % d.Th. dismutierten Ethylaminalester.

## Patentansprüche

1. Verfahren zur Herstellung von ortho-Amiden der Formel 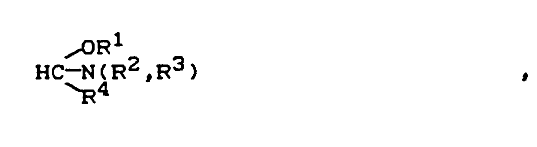 in der
R¹ geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₂-C₈-Alkylen-OM¹ oder C₇-C₁₀-Aralkyl bedeutet,
R² und R³ unabhängig voneinander geradkettiges oder verzweigtes C₁-C₈-Alkyl, C₂-C₈-Alkenyl, C₂-C₈-Alkoxyalkyl, C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R² und R³ gemeinsam mit dem N-Atom, das sie substituieren, einen 5- bis 8-gliedrigen Ring bilden können, der ein weiteres Heteroatom aus der Gruppe N, O und S enthalten kann, und
R⁴ für -OR⁵ oder -N(R⁵,R⁶) steht, worin R⁵ und R⁶ unabhängig voneinander und unabhängig von R² und R³ den für R² und R³ genannten Bedeutungsumfang haben,
dadurch gekennzeichnet, daß man Salze der Formel 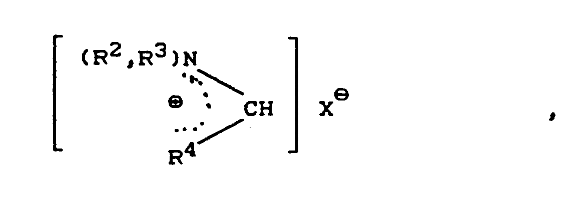 in der
R², R³ und R⁴ die genannte Bedeutung haben und
X^{⊖} das C₁-C₈-Alkylsulfatanion, das C₆-C₁₂-Arylsulfonatanion, das Tetrafluoroboranatanion, das C₆-C₁₂-Arylsulfatanion, das Chloridanion, das Bromidanion, das Iodidanion, das Hexafluorophosphatanion, das C₁-C₈-Alkylsulfonatanion, das C₁-C₈-Halogenalkylsulfonatanion, das C₁-C₈-Halogenalkylsulfatanion, das Perchloratanion oder das Hexachloroantimonatanion bedeutet,
in Gegenwart von Alkoholaten der Formel
M¹OR¹,
in der
R¹ den genannten Bedeutungsumfang besitzt und
M¹ ein Äquivalent eines Alkali- oder Erdalkalimetallkations ist,
in einem Nichtlösungsmittel umsetzt, wobei eine Alkoholat-Suspension in demselben Nichtlösungsmittel eingesetzt wird, die durch Behandlung von in einem Alkohol gelösten Alkoholat in höhersiedenden Nicht-Lösungsmitteln bei einer Temperatur von 120 bis 200°C, bevorzugt 130 bis 180°C und destillativer. Entfernung des gesamten Alkohols und Kristallalkohols erhalten worden ist.

2. Verfahren nach Anspruch 1, dadurch gekennzeichnet, daß man Salze der Formel 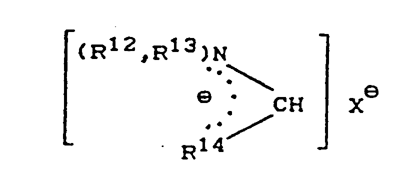 umsetzt, in der
R¹² und R¹³ unabhängig voneinander geradkettiges oder verzweigtes C₁-C₈-Alkyl, Cyclopropyl, Cyclopentyl, Cyclohexyl, Phenyl oder Benzyl bedeuten und R¹² und R¹³ weiterhin gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann,
R¹⁴ für -OR¹² oder -N(R¹²,R¹³) steht und
X^{⊖} das C₁-C₈-Alkylsulfatanion, das C₆-C₁₂-Arylsulfonatanion, das Tetrafluoroboranatanion, das C₆-C₁₂-Arylsulfatanion, das Chloridanion, das Bromidanion, das Iodidanion, das Hexafluorophosphatanion, das C₁-C₈-Alkylsulfonatanion, das C₁-C₈-Halogenalkylsulfonatanion, das C₁-C₈-Halogenalkylsulfonatanion, das Perchloratanion oder das Hexachloroantimonatanion bedeutet.

3. Verfahren nach Anspruch 1, dadurch gekennzeichnet, daß man Salze der Formel 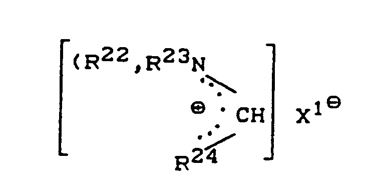 umsetzt, in der
R²² und R²³ unabhängig voneinander geradkettiges oder verzweigtes C₁-C₄-Alkyl bedeuten und R²² und R²³ weiterhin gemeinsam mit dem N-Atom, das sie substituieren, Morpholino, Pyrrolidino oder Piperidino bedeuten können,
R²⁴ für -OR²² oder -N(R²²,R²³) steht und
X^{1⊖} das C₁-C₈-Alkylsulfatanion, das Chloridanion, das Bromidanion oder das Iodidanion bedeutet.

4. Verfahren nach Anspruch 1, dadurch gekennzeichnet, daß man Salze der Formel 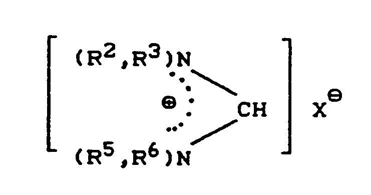 einsetzt, in der
R², R³, R⁵, R⁶ und X^{⊖} die in Anspruch 1 angegebene Bedeutung haben.

5. Verfahren nach Anspruch 4, dadurch gekennzeichnet, daß man Salze der Formel 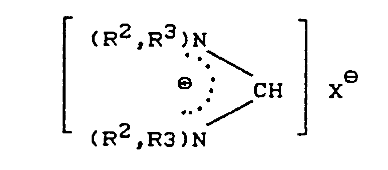 einsetzt, in der
R², R³ und X^{⊖} die in Anspruch 1 angegebene Bedeutung haben.

6. Verfahren nach Anspruch 1, dadurch gekennzeichnet, daß in Gegenwart von Alkoholaten der Formel
M²OR¹¹ (VI)
gearbeitet wird, in der
R¹¹ für geradkettiges oder verzweigtes C₁-C₅-Alkyl, C₂-C₅-Alkoxyalkyl oder C₂-C₄-Alkylen-OM² steht und
M² das Natrium- oder Kaliumkation bedeutet.

7. Verfahren nach Anspruch 6, dadurch gekennzeichnet, daß in Gegenwart von Alkoholaten der Formel
M²OR²¹ (VII)
gearbeitet wird, in der
R²¹ für geradkettiges oder verzweigtes C₁-C₅-Alkyl steht und
M² das Natrium- oder Kaliumkation bedeutet.

8. Verfahren nach Anspruch 1, dadurch gekennzeichnet, daß das Alkoholat Methylat ist.

9. Verfahren nach Anspruch 1, dadurch gekennzeichnet, daß das Verhältnis Salz : Alkoholat von 1,5:1 bis 1:1,5, bevorzugt von 1,2:1 bis 1:1,2 liegt.

## Claims

1. Process for the preparation of ortho-amides of the formula 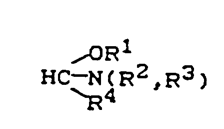 in which
R¹ denotes straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₂-C₈-alkylene-OM¹ or C₇-C₁₀-aralkyl,
R² and R³, independently of one another, represent straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₆-C₁₂-aryl, C₇-C₁₀-aralkyl, or a 5- to 8-membered saturated or unsaturated heterocyclic ring whose hetero atoms 1 or 2 are from the group comprising N, O and S, where furthermore R² and R³, together with the N atom which they substitute, can form a 5- to 8-membered ring which can contain a further hetero atom from the group comprising N, O and S, and
R⁴ represents -OR⁵ or -N(R⁵,R⁶) in which R⁵ and R⁶, independently of one another and independently of R² and R³, have the range of meanings mentioned in the case of R² and R³,
characterised in that salts of the formula in which 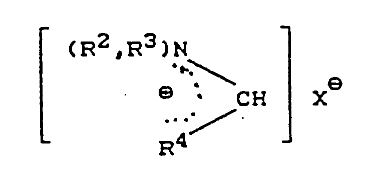
R², R³ and R⁴ have the abovementioned meaning and
X^{⊖} denotes the C₁-C₈-alkylsulphate anion, the C₆-C₁₂-arylsulphonate anion, the tetrafluoroboranate anion, the C₆-C₁₂-arylsulphate anion, the chloride anion, the bromide anion, the iodide anion, the hexafluorophosphate anion, the C₁-C₈-alkylsulphonate anion, the C₁-C₈-halogenoalkylsulphonate anion, the C₁-C₈-halogenoalkylsulphate anion, the perchlorate anion or the hexachloroantimonate anion,
are reacted in a non-solvent in the presence of alcoholates of the formula
M¹OR¹
in which
R¹ has the range of meanings mentioned and
M¹ is an equivalent of an alkali metal cation or alkaline earth metal cation,
an alcoholate suspension in the same non-solvent being employed, which has been obtained by treating an alcoholate, dissolved in alcohol, in higher-boiling non-solvents at a temperature of 120 to 200°C, preferably 130 to 180°C, and removing the entire alcohol and alcohol of crystallisation by distillation.

2. Process according to Claim 1, characterised in that salts of the formula 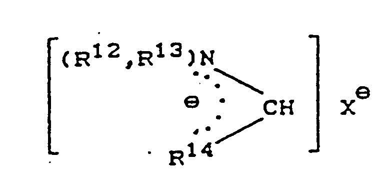 are reacted, in which
R¹² and R¹³, independently of one another, denote straight-chain or branched C₁-C₈-alkyl, cyclopropyl, cyclopentyl, cyclohexyl, phenyl or benzyl and R¹² and R¹³ furthermore, together with the N atom which they substitute, can form a 5-to 8-membered saturated or unsaturated N-heterocyclic ring which can contain a further hetero atom from the group comprising N, O and S,
R¹⁴ represents -OR¹² or -N(R¹²,R¹³) and
X^{⊖} denotes the C₁-C₈-alkylsulphate anion, the C₆-C₁₂-arylsulphonate anion, the tetrafluoroboranate anion, the C₆-C₁₂-arylsulphate anion, the chloride anion, the bromide anion, the iodide anion, the hexafluorophosphate anion, the C₁-C₈-alkylsulphonate anion, the C₁-C₈-halogenoalkylsulphonate anion, the C₁-C₈-halogenoalkylsulphate anion, the perchlorate anion or the hexachloroantimonate anion.

3. Process according to Claim 1, characterised in that salts of the formula 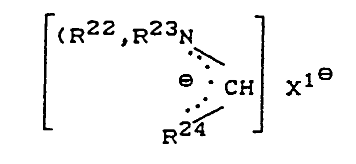 are reacted, in which R²² and R²³, independently of one another, denote straight-chain or branched C₁-C₄-alkyl and R²² and R²³ furthermore, together with the N atom which they substitute, can denote morpholino, pyrrolidino or piperidino,
R²⁴ represents -OR²² or -N(R²²,R²³) and
X^{1⊖} denotes the C₁-C₈-alkylsulphate anion, the chloride anion, the bromide anion or the iodide anion.

4. Process according to Claim 1, characterised in that salts of the formula 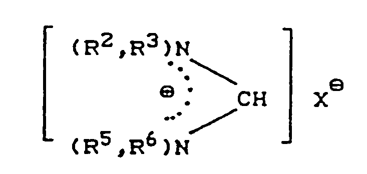 are employed, in which
R², R³, R⁵, R⁶ and X^{⊖} have the meaning given in Claim 1.

5. Process according to Claim 4, characterised in that salts of the formula 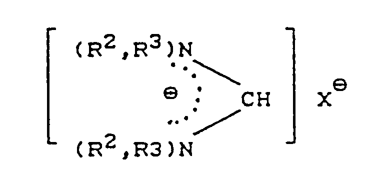 are employed, in which
R², R³ and X^{⊖} have the meaning given in Claim 1.

6. Process according to Claim 1, characterised in that the process is carried out in the presence of alcoholates of the formula
M²OR¹¹ (VI)
in which
R¹¹ represents straight-chain or branched C₁-C₅-alkyl, C₂-C₅-alkoxyalkyl or C₂-C₄-alkylene-OM² and
M² denotes the sodium or potassium cation.

7. Process according to Claim 6, characterised in that the process is carried out in the presence of alcoholates of the formula
M²OR²¹ (VII)
in which
R²¹ represents straight-chain or branched C₁-C₅-alkyl and
M² denotes the sodium or potassium cation.

8. Process according to Claim 1, characterised in that the alcoholate is methylate.

9. Process according to Claim 1, characterised in that the ratio of salt : alcoholate is from 1.5:1 to 1:1.5, preferably 1.2:1 to 1:1.2.

## Revendications

1. Procédé pour la préparation d'ortho-amides répondant à la formule 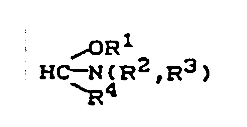 dans laquelle
R¹ représente un groupe alkyle en C₁-C₈ à chaîne droite ou ramifiée, un groupe alcényle en C₂-C₈, un groupe alcoxyalkyle en C₂-C₈, un groupe alcoxyalcényle en C₃-C₈, un groupe cycloalkyle en C₃-C₈, un groupe alkylène(en C₂-C₈)-OM¹ ou un groupe aralkyle en C₇-C₁₀,
R² et R³ représentent, indépendamment l'un de l'autre, un groupe alkyle en C₁-C₈ à chaîne droite ou ramifiée, un groupe alcényle en C₂-C₈, un groupe alcoxyalkyle en C₂-C₈, un groupe alcoxyalcényle en C₃-C₈, un groupe cycloalkyle en C₃-C₈, un groupe aryle en C₆-C₁₂, un groupe aralkyle en C₇-C₁₀ ou encore un noyau hétérocyclique penta- à octogonal saturé ou insaturé dont les hétéroatomes représentent un ou deux atomes choisis parmi le groupe comprenant N, O et S, dans lesquels, en outre, R² et R³ peuvent former, ensemble avec l'atome N qu'ils substituent , un noyau penta- à octogonal qui peut contenir un autre hétéroatome choisi parmi le groupe comprenant N, O et S, et
R⁴ représente -OR⁵ ou -N(R⁵,R⁶) où R⁵ et R⁶ ont, indépendamment l'un de l'autre et indépendamment de R² et R³, la portée de signification mentionnée pour R² et R³,
caractérisé en ce qu'on fait réagir des sels de formule 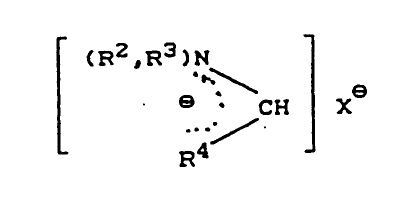 dans laquelle
R², R³ et R⁴ ont la signification mentionnée, et
X^{⊖} représente l'anion d'alkyl(en C₁-C₈)sulfate, l'anion d'aryl(en C₆-C₁₂)sulfonate, l'anion de tétrafluoroboranate, l'anion d'aryl(en C₆-C₁₂)sulfate, l'anion chlorure, l'anion bromure, l'anion iodure, l'anion d'hexafluorophosphate, l'anion d'alkyl(en C₁-C₈)sulfonate, l'anion d'halogénalkyl(en C₁-C₈)sulfonate, l'anion d'halogénalkyl(en C₁-C₈)sulfate, l'anion perchlorate ou encore l'anion d'hexachloroantimonate,
en présence d'alcoolates répondant à la formule
M¹OR¹,
dans laquelle
R¹ possède la portée de signification mentionnée et
M¹ est un équivalent d'un cation de métal alcalin ou de métal alcalino-terreux,
dans un non-solvant, dans lequel on met en oeuvre une suspension d'alcoolate dans le même non-solvant, que l'on a obtenue par traitement d'un alcoolate dissous dans un alcool dans des non-solvants à point d'ébullition très élevé à une température de 120 à 200°C, de préférence de 130 à 180°C, et par élimination par distillation de l'alcool global et de l'alcool de cristallisation.

2. Procédé selon la revendication 1, caractérisé en ce qu'on fait réagir des sels de formule 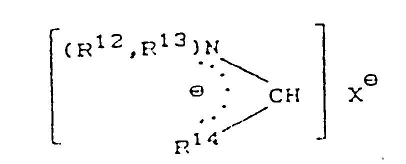 dans laquelle
R¹² et R¹³ représentent, indépendamment l'un de l'autre, un groupe alkyle en C₁-C₈ à chaîne droite ou ramifiée, un groupe cyclopropyle, un groupe cyclopentyle, un groupe cyclohexyle, un groupe phényle ou un groupe benzyle, R¹² et R¹³ pouvant former en outre, ensemble avec l'atome N qu'ils substituent , un noyau N-hétérocyclique penta- à octogonal saturé ou insaturé qui peut contenir un autre hétéroatome choisi parmi le groupe comprenant N, O et S,
R¹⁴ représente -OR¹² ou -N(R¹²,R¹³), et
X^{⊖} représente l'anion d'alkyl(en C₁-C₈)sulfate, l'anion d'aryl(en C₆-C₁₂)sulfonate, l'anion de tétrafluoroboranate, l'anion d'aryl(en C₆-C₁₂)sulfate, l'anion chlorure, l'anion bromure, l'anion iodure, l'anion d'hexafluorophosphate, l'anion d'alkyl(en C₁-C₈)sulfonate, l'anion d'halogénalkyl(en C₁-C₈)sulfonate, l'anion d'halogénalkyl(en C₁-C₈)sulfate, l'anion perchlorate ou encore l'anion d'hexachloroantimonate.

3. Procédé selon la revendication 1, caractérisé en ce qu'on fait réagir des sels de formule 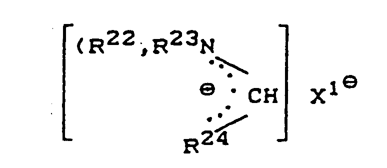 dans laquelle
R²² et R²³ représentent, indépendamment l'un de l'autre, un groupe alkyle en C₁-C₄ à chaîne droite ou ramifiée, R²² et R²³ pouvant former en outre, ensemble avec l'atome N qu'ils substituent , un groupe morpholino, un groupe pyrrolidino ou un groupe pipéridino,
R²⁴ représente -OR²² ou -N(R²²,R²³), et
X^{1⊖} représente l'anion d'alkyl(en C₁-C₈)sulfate, l'anion chlorure, l'anion bromure ou l'anion iodure.

4. Procédé selon la revendication 1, caractérisé en ce qu'on met en oeuvre des sels de formule 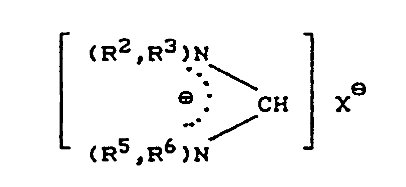 dans laquelle R², R³, R⁵, R⁶ et X^{⊖} ont la signification indiquée à la revendication 1.

5. Procédé selon la revendication 4, caractérisé en ce qu'on met en oeuvre des sels de formule 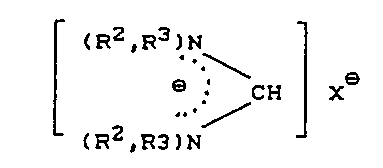 dans laquelle
R², R³ et X^{⊖} ont la signification indiquée à la revendication 1.

6. Procédé selon la revendication 1, caractérisé en ce qu'on travaille en présence d'alcoolates répondant à la formule
M²OR¹¹ (VI)
dans laquelle
R¹¹ représente un groupe alkyle en C₁-C₅ à chaîne droite ou ramifiée, un groupe alcoxyalkyle en C₂-C₅ ou un groupe alkylène(en C₂-C₄)-OM², et
M² représente le cation du sodium ou du potassium.

7. Procédé selon la revendication 6, caractérisé en ce qu'on travaille en présence d'alcoolates répondant à la formule
M²OR²¹ (VII)
dans laquelle
R²¹ représente un groupe alkyle en C₁-C₅ à chaîne droite ou ramifiée, et
M² représente le cation du sodium ou du potassium.

8. Procédé selon la revendication 1, caractérisé en ce que l'alcoolate est le méthylate.

9. Procédé selon la revendication 1, caractérisé en ce que le rapport sel:alcoolate se situe de 1,5:1 à 1:1,5, de préférence de 1,2:1 à 1:1,2.
